# EUROPEAN PATENT APPLICATION

(11) **EP 2 327 781 A1**
(43) Date of publication of application: **01.06.2011**
(21) Application number: 09014783.6
(22) Date of filing: 27.11.2009
(51) Int. Cl.: C12N 15/11, A61K 31/7088, A61K 35/00

(54) **Micro-RNA and tissue repair**

(71) Applicant: RWTH Aachen, 52056 Aachen (DE)
(72) Inventor: Weber, Christian, 52074 Aachen (DE); Zernecke, Alma, 52074 Aachen (DE); Bidzhekov, Kiril, 52074 Aachen (DE)
(74) Representative: Kröncke, Rolf

(57) **Abstract**

The present invention relates to the use of miR-126 in postnatal tissue repair. In particular, the isolated nucleic acids comprising the miR-126 nucleic acid sequence are useful in the prophylaxis or treatment of atherosclerosis as well as for the prophylaxis or treatment of tissue damages resulting in or due to various arterial based diseases. In a further aspect, the present invention relates to the use of nucleic acids comprising the miR-126 nucleic acid sequence for the recruitment of progenitor cells to tissue, in particular, blood vessels.

## Description

The present invention relates to the use of mIR-126 in postnatal tissue repair. In particular, the isolated nucleic acids comprising the miR-126 nucleic acid sequence are useful in the prophylaxis or treatment of atherosclerosis as well as for the prophylaxis or treatment of tissue damages resulting in or due to various arterial based diseases. In a further aspect, the present invention relates to the use of nucleic acids comprising the miR-126 nucleic acid sequence for the recruitment of progenitor cells to tissue, in particular, blood vessels.

### Prior art

Tissue damages, in particular damages of blood vessels, like damages in arteries and venes, are the cause for many wide spread diseases of individuals eventually resulting in death. For example, atherosclerosis also known as Arteriosclerotic Vascular Disease or ASVD is the condition in which an artery wall thickens as the result of a build-up of fatty materials such as cholesterol. Atherosclerosis affects arterial blood vessels, a chronic inflammatory response in the walls of arteries, said chronic inflammatory response is due to the accumulation of macrophage white blood cells and promoted by LDL. Atherosclerosis is caused by the formation of multiple plaques in the arteries. Atherosclerosis produces two main problems, firstly, the plaques eventually lead to plaque ruptures and clots inside the artery lumen over the ruptures, thus, resulting in an insufficient blood supply to the tissues and organ it feeds. Secondly, if the compensating artery enlargement process is excessive, then an aneurysm results. The plaques occurring in atherosclerosis may rupture, thus, causing the formation of a thrombus which eventually results in an infarction. One of the most common recognized scenarios is called coronary thrombosis of a coronary artery causing myocardial infarction. Even worse it is the same process in an artery to the brain, commonly called stroke.

Another common scenario in very advanced forms of disease is claudication from insufficient blood supply to the legs, typically due to a combination of both stenosis and aneurysmal segments narrowed with clots, e.g. arterial obstructive disease. Since atherosclerosis is a body-wide process, similar events occur also in the arteries to the brain, intestines, kidneys, legs, etc.

Today, only the symptoms of atherosclerosis are treated, for example symptoms such as angina pectoris etc. Moreover, the risk factors for atherosclerosis may be determined. For example dietary changes may benefit as well as stop smoking etc.

Further, surgical interventions may be conducted, for example, minimally invasive angioplasty procedures that may include stents to physically expand narrow arteries and major invasive surgery, such as bypass surgery.

However, there is still an ongoing need for means allowing prophylaxis or treatment of atherosclerosis as well as for postnatal tissue repair in general.

Cell death through apoptosis is a process of ubiquitous importance in embryonic development but also for cell homeostasis in adults, and has been implicated in many diseases including atherosclerosis. In the course of apoptotic programs, a series of molecular events culminates in DNA fragmentation and blebbing of the cell membrane accompanied by the release of small membranous particles from its surface, termed apoptotic bodies (AB). Endothelial cell-derived microparticles released after apoptotic stimuli can originate from atherosclerotic plaques and their levels in the circulation have been associated with endothelial dysfunction and arterial stiffness, indicating that they may serve as a prognostic marker for atherosclerotic vascular disease. This release has been viewed as an attempt to reverse or escape cell death by removing parts of the caspase machinery and to elicit eat-me signals attracting phagocytes for cell elimination, but a function in paracrine signalling to neighbouring cells for modulating immune-cell responses or repair mechanisms has not been fully elucidated. While AB can induce proliferation and differentiation of endothelial progenitor cells in vitro implying a role angiogenesis, they can also be engulfed by phagocytes triggering secretion of cytokines of growth factors, namely vascular-endothelial growth factor (VEGF). After DNA damage, hypoxia, or in the context of arterial injury, the chemokine CXCL12 has been implicated In the recruitment of progenitor cells for tissue repair or angiogenesis via its receptor CXCR4 and in the defense and survival programs counteracting process related to apoptosis.

MicroRNAs (miRNAs or miR) are a class of single-stranded non-coding RNAs (ncRNAs) that have been conserved In evolution from plants to animals. Mature miRNAs are typically around 17-24 nucleotides in length, but may be longer or shorter. They are generated in cells from miRNA precursors as the result of a series of RNA processing steps. First a pri-miRNA transcript having a hairpin structure is produced. The mature miRNA is located within one arm/strand of this precursor hairpin (the opposite strand of the hairpin, known as the star(*) strand, is generally degraded, see Wang et al, 2008, Dev. Cell, 15, p 261-271). The pri-miRNA is processed in the nucleus to form a pre-miRNA which is exported to the cytoplasm. The pre-miRNA undergoes further processing in the cytoplasm to form the mature miRNA. It is the mature miRNA that inhibits expression of its target gene at the post- transcriptional level by binding to the mRNA of the target gene by Watson-Crick base pairing. miRNAs have been found to have roles in a variety of biological processes including developmental timing, differentiation, apoptosis, cell proliferation, organ development, and metabolism.

In WO 2008/073921, methods and composition for identifying genes or genetic pathways modulated by miR-126, using miR-126 to modulate a gene or a gene pathway, using this profile in assessing the condition of a patient and/or treating the patient with an appropriate miRNA are disclosed. That is, said document recognizes that miR-126 may be used in the treatment of cancer for inhibiting proliferation in lung cancer cell lines as well as inhibiting tumor growth of lung cancer cell lines in mice.

WO 2009/126650 discloses the use of miR-126 and inhibitors of miR-126 for modulating angiogenesis. In particular, said patent application provides inhibitors of miR-126 for inhibition of angiogenesis in mice.

At present, it is generally accepted that miR-126 is responsible for excessive angiogenesis in higher dosage and, consequently, inhibitors of miR-126 should be used to modulate excessive angiogenesis as well as formation and repair of blood vessels.

Herein, the present inventors recognized that apoptotic bodies and, in particular, components present in apoptotic bodies, namely miR-126, are able to promote postnatal tissue repair and, in addition to act prevent the development of as well as being useful for the treatment of atherosclerosis and other forms of tissue damage, Moreover, it is demonstrated herein, that miR-126 is able to contribute to homeostasis in health and disease requiring progenitor cell influx.

### Brief description of the present invention

Hence, in the first aspect, the present invention relates to an isolated nucleic acid comprising a miR-126 nucleic acid sequence for use in postnatal tissue repair or for use in the prophylaxis of tissue damage.

The present inventors recognized that miR-126 nucleic acid sequences promotes tissue regeneration and tissue repair by e.g. mobilizing and recruiting progenitor cells, thus, e.g. limiting plaque formation and atheroprogression.

In a further aspect, the present invention relates to isolated nucleic acids comprising a miR-126 nucleic acid sequence for use in the prophylaxis or treatment of atherosclerosis. In particular, said nucleic acid sequence may be used for the prophylaxis or treatment of tissue damages resulting in or due to coronary heart disease, like myocardial infarction, stroke, peripheral artery disease, aneurysm formation but also disease related to endothelial and vascular dysfunction such as pulmonary hypertension, transplant arteriopathy or various forms of vasculitis.

In another aspect, the present invention relates to apoptotic bodies, in particular, endothelial derived apoptotic bodies useful in the prophylaxis or treatment of postnatal tissue repair, in particular useful in the prophylaxis or treatment of atherosclerosis. In particular, said apoptotic bodies are present in isolated form either from natural or from synthetic sources, contain miR-126 nucleic acid sequence. The apoptotic bodies as well as pharmaceutical compositions comprising the nucleic acid sequence according to the present invention or the apoptotic bodies according to the present invention are adapted for intravenous administration, or biofunctionalized (e.g. antibody-tagged) artificial particles such as polybutylcyanoacrylat (PBCA)-stabilized microbubbles containing miR-126 or mimics for high-frequency ultrasound-mediated imaging and delivery, or cholesterol or other lipid-tagged forms of miR-126 as e.g. described for antagomirs for targeted delivery.

### Brief description of the drawings

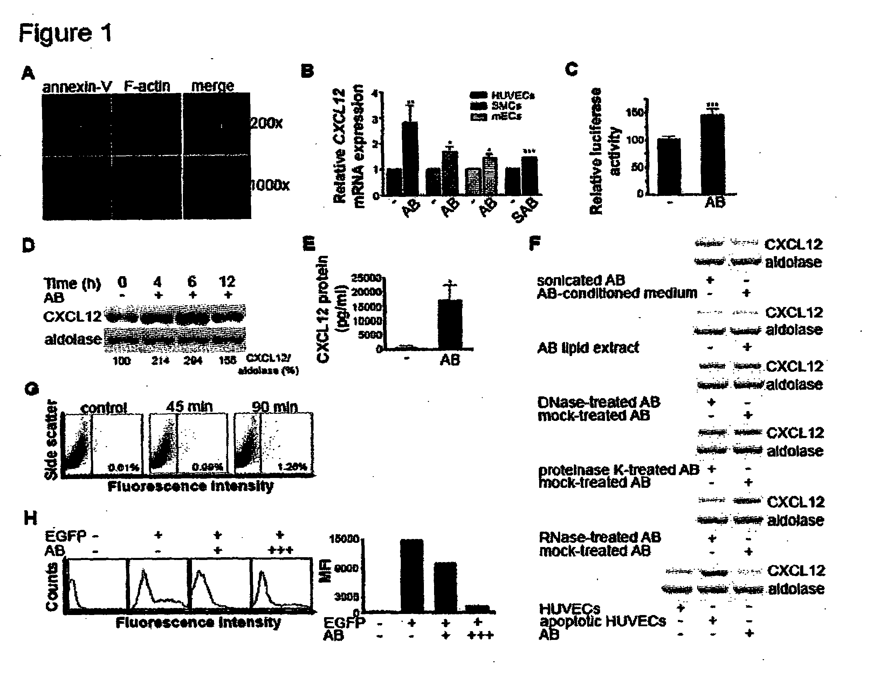

**Figure 1. AB confer functional RNA to Induce CXCL12 In endothelial cells;** A, Confocal microscopy of F-actin⁺ fibers (red) in HUVECs incubated with annexin-V⁺ AB (green). **B,** Real-time PCR of *CXCL12* expression in HUVECs (n=6), SMCs (n=2), mouse ECs (mECs, n=3) after treatment with/without AB or in HUVECs (n=2) treated with/without SMC-AB (SAB). C, *CXCL12*-luciferase promoter activity in HUVECs after treatment with/without AB (n=4). **D,** Representative RT-PCR analysis of the abundance of *CXCL12* mRNA expression in HUVECs treated with/without AB for indicated periods. **E,** CXCL12 protein secretion from HUVECs treated with/without AB (n=3). **F,** Representative RT-PCR analysis of the abundance of *CXCL12* mRNA expression in AB, apoptotic HUVECs, and HUVECs incubated with/without AB-conditioned medium, AB-lipid extracts, or sonicated AB pretreated as indicated (n=3). **G,** Flow cytometry analysis of HUVECs treated with/without AB from FITC⁺ oligonucleotide-transfected HUVECs for Indicated periods, H, and of pEGFP-transfected HUVECs treated with/without AB (1x or 3x) from HUVECs transfected with pEGFP-silencing siRNA. Data represent mean ± SEM. **p*<0.05, ***p*<0.01, ****p*<0.005.

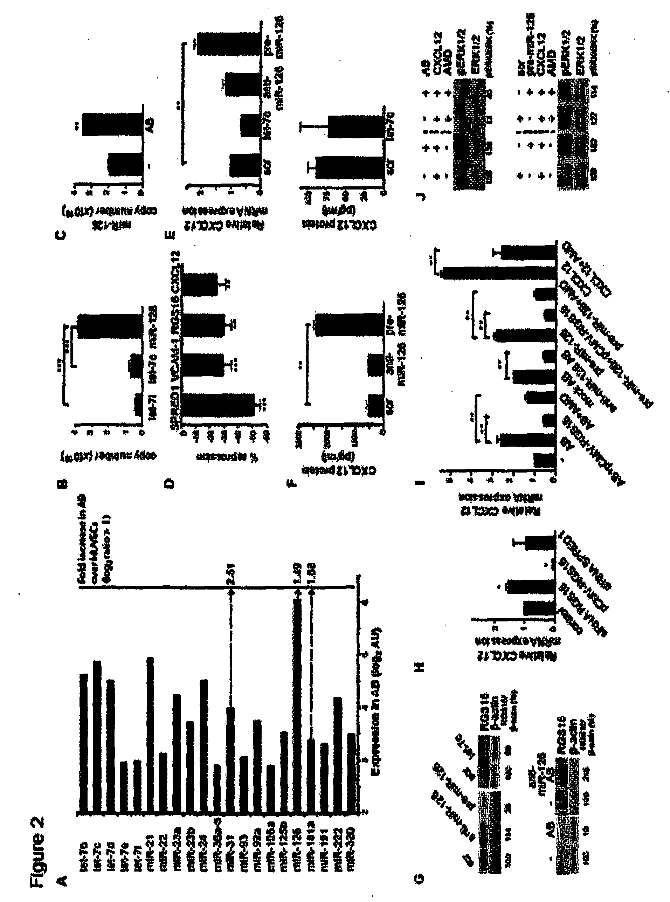

**Figure 2****. AB carry miR-126 to repress RGS16 and to enhance CXCR4 signals; A,** Profile of miR expressed in AB with >4-fold increase in abundance over baseline (log₂>2) and ratios for miR enriched with >2-fold increase in abundance over baseline (log₂>1) in AB vs. HUVECs (right column) as analyzed by bioarray-chips (AU = arbitrary units). **B,** Real-time PCR of selected miR expression (white bars, **A),** relative to *U6*. **C,** Relative miR-126 expression in HUVECs treated with/without AB for 1 hour (n=3). **D**, Repression of luciferase activity in HUVECs co-transfected with pro-miR-126 and pMIR-Report plasmids carrying miR-126-binding sites found in the 3'UTR of indicated target genes downstream of a luciferase reporter (relative to empty pMIR-control; n=3). **E,F** The abundance of *CXCL12* mRNA and CXCL12 protein expression in HUVECs transfected with pro-miR-126, anti-miR-126, let-7c, or scrambled control sequences (scr), as determined by real-time PCR and ELISA (n=2-5). **G,** RGS16 protein expression in HUVECs transfected with pro-miR-126, anti-miR-126, let-7c, or scr or treated with/without AB from anti-miR-126-or mock-transfected HUVECs compared to B-actin (densiometric data of n=2 to 5 experiments). H,I Real-time PCR of *CXCL12* mRNA expression in HUVECs transfected with siRNA against *RGS16* or *SPRED1,* or pCMV-RGS16, relative to mock-sequence controls (H), and in HUVECs treated with/without AB from anti-miR-126- or mock-transfected HUVECs, CXCL12 or AMD3100, or transfected with pro-miR-126 and pCMV-RGS16 (I), as indicated (n=2-5). **J**, Immunoblot detection of total ERK1/2 and CXCL12-induced phosphorylation (P-ERK1/2) in HUVECs treated with AB or AMD3100 or transfected with pro-miR-126 or scr-miRNA (densiometric data of n=3 experiments). Data represent mean ± SEM. **p*<0.05, ***p*<0.005, ****p*<0,001.

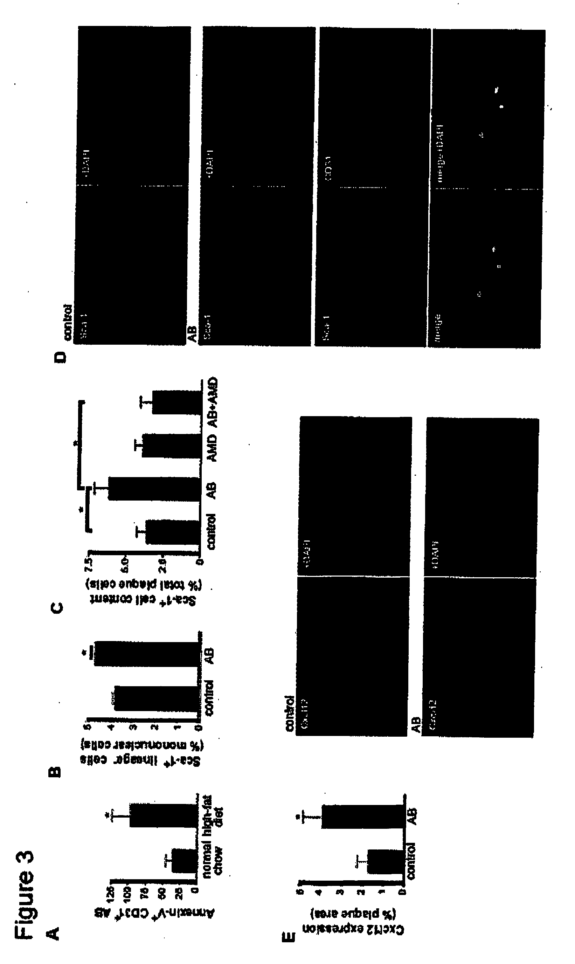

**Figure 3****. Mobilization of progenitor cells and CXCL12 induction by AB *in vivo*;** A, Number of circulating AB in *Apoe*^{*-*/*-*} mice on normal chow or high-fat diet (n=5), and **B,** of circulating Sca-1⁺ lineage⁻ progenitor cells after injection of PBS (control) or AB, determined by flow cytometry (n=5). **C-E,** *Apoe^{-l-}* mice were fed a high-fat diet for 6 weeks, injected twice weekly with PBS or AB, and treated with or without AMD3100 (n=7-8). **C**, Quantification of Sca-1⁺ cells and **D**, representative images of double-immunofluorescence staining for Sca-1 and CD31 in the aortic root of control or AB-treated *Apoe*^{-/-} mice. **E,** Quantification and representative images of immunofluorescence of Cxcl12 In aortic root plaques; nuclei are stained by DAPI. Data represent mean ± SEM.**p*<0.05, ***p*<0.01.

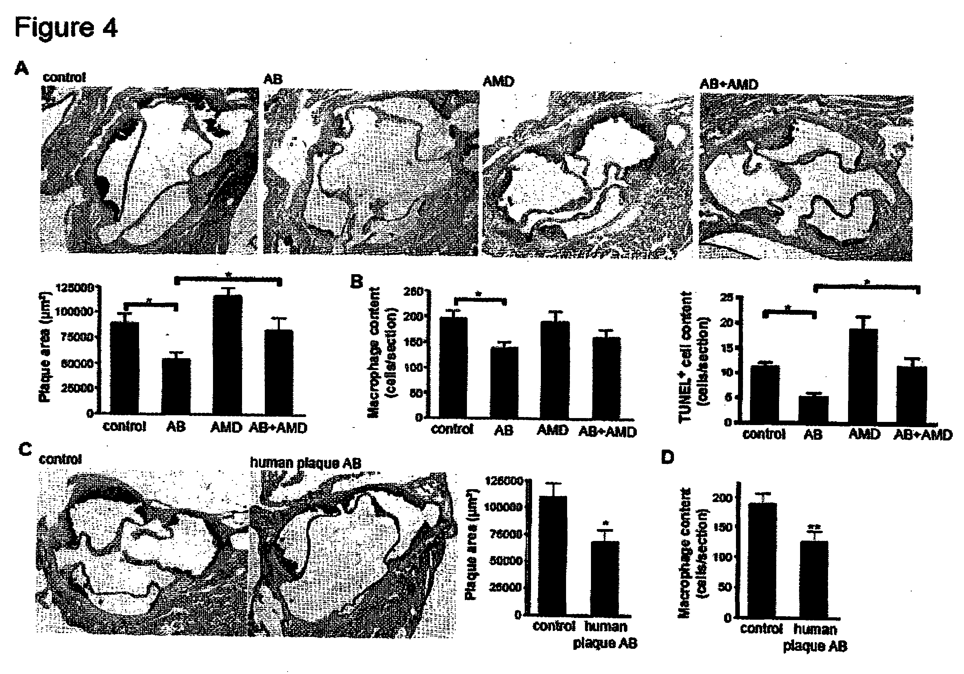

**Figure 4****. AB protect against early atherosclerotic lesion formation; A,B,** *Apoe*^{-/-} mice fed a high-fat diet for 6 weeks and injected twice weekly with PBS or AB, and treated with/without AMD3100 (n=7-8). **A,** Representative images of aortic roots and quantification of Oil-red-O⁺ atherosclerotic lesion area and **B,** quantification of plaque macrophage and TUNEL⁺ apoptotic cell content. **C,D**, *Apoe*^{-/-} mice fed a high-fat diet for 6 weeks and injected once weekly with PBS or human plaque-derived AB (n=7-9). **C,** Representative images and quantification of Oil-red-O⁺ atherosclerotic plaque area and **D,** macrophage content. Data represent mean ± SEM. **p*<0.05, ***p*<0.01

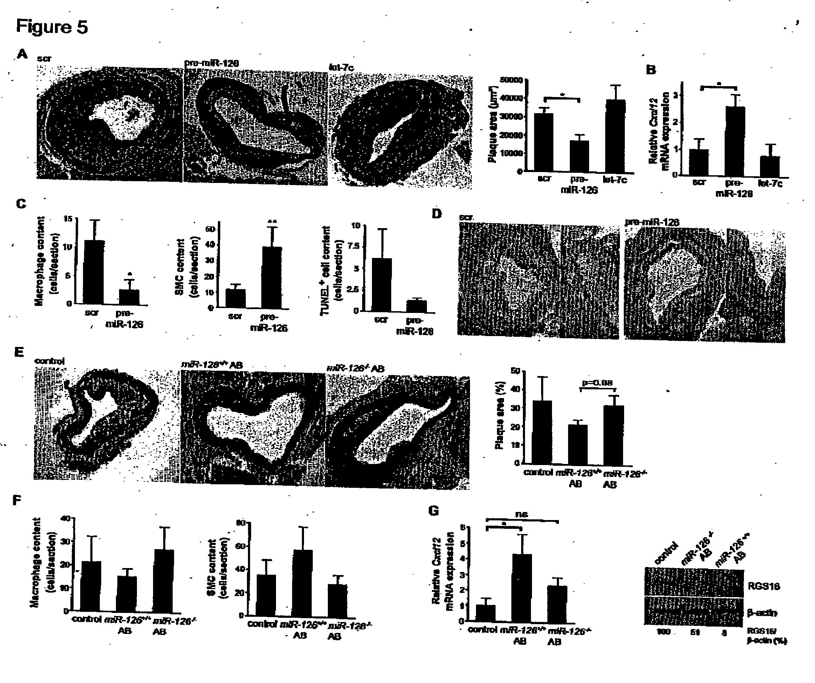

**Figure 5****. Transfer of miR-126 or AB carrying miR-126 reduces plaque formation; (A to D)** Collar-induced plaque formation in carotid arteries of *Apoe*^{*-*/*-*} mice 4 weeks after transduction with scrambled control (scr), pro-miR-126, or let-7c (n=7-8). **A**, Representative images and quantification of pentachrome-stained plaque area, and **B**, *Cxcl12* mRNA expression relative to *18s* rRNA in carotid arteries, as determined by real-time PCR. **C**, Quantification of plaque macrophage, TUNEL⁺ apoptotic cell and SMC content, and **D**, representative images of Sirius-red staining for collagen. Data represent mean ± SEM. **p*<0.05, ***p*<0.01. **E-G,** Carotid arteries of *Apoe*^{-/-} mice on a high-fat diet for 10 weeks were intraluminally incubated with/without AB isolated from *miR-126*^{+/+} or *miR-126*^{-/-} mice 5 weeks before sacrifice (n=5-6). **E,** Representative images and quantification of pentachrome-stained plaque area, and **F**, macrophage and SMC content. **G**, Rgs16 protein expression compared to β-actin and *Cxcl12* mRNA relative to *18s* rRNA expression are shown. Data represent mean ± SEM. **p*<0.05, ***p*<0.01.

### Detailed description of the present invention

The present invention provides isolated nucleic acid molecules comprising a miR-126 nucleic acid sequence for use in postnatal tissue repair. That is, the present invention is related to a new use of micro RNA known as miR-126. Mature miR-126 is derived from one strand of its hairpin precursor, while miR-126* (a distinct sequence) is derived from the opposite strand of the same hairpin precursor (as illustrated in Wang et al., 2008, Dev. Cell, 15, p 261-271, the contents of which are hereby Incorporated by reference).

As used herein, the term "miR-126" is used to refer to a miRNA having at least the sequence UCGUACCGUGAGUAAUAAUGCG (22 nucleotides, SEQ ID NO. 1) or UCGUACCGUGAGUAAUAAUGC (SEQ ID NO. 2, which comprises the first 21 nucleotides of SEQ ID No. 1), and to homologues thereof. A "miR-126 nucleic acid sequence" includes the full length precursor of miR-126, or complement thereof or processed (i.e., mature) sequence of miR-126 and related sequences set forth herein, as well as 5, 6, 7, 8, 9, 10, 11, 12, 13, 14, 15, 16, 17, 18, 19, 20, 21, 22, 23, 24, 25, 20, 27, 28, 29 or more nucleotides of a precursor miRNA or its processed sequence, or complement thereof, including all ranges and integers there between. In certain embodiments, the miR-126 nucleic acid sequence contains the full-length processed miRNA sequence or complement thereof and is referred to as the "miR- 126 full-length processed nucleic acid sequence". In still further aspects, the miR-126 nucleic acid comprises at least one 5, 6, 7, 8, 9, 10, 11, 12, 13, 14, 15, 16, 17, 18, 19, 20, 21, 22, 23, 24, 25, 50 nucleotide (including all ranges and integers there between) segment or complementary segment of a miR-126 that is at least 75, 80, 85, 90, 95, 98, 99 or 100% identical to SEQ ID NO:1 to SEQ ID NOs 1 or 2. The general term miR-126 includes all members of the miR-126 family that share at least part of a mature miR-126 sequence. Mature miR-126 sequences include hsa-miR-126 UCGUACCGUGAGUAAUAAUGC (MIMAT0000445, SEQ ID1); hsa-miR-126* CAUUAUUACUUUUGGUACGCG (MIMAT0000444, SEQ ID5); bta-miR-126 CGUACCGUGAGUAAUAAUGCG (MIMAT0003540, SEQ ID6); dre-miR-126 UCGUACCGUGAGUAAUAAUGC (MIMAT0001823, SEQ ID7); dre-miR-126* CAUUAUUACUUUUGGUACGCG (MIMAT0003157, SEQ ID8); fru-miR-128 UCGUACCGUGAGUAAUAAUGC (MIMAT0002957, SEQ ID9); mmu-miR-128-5p CAUUAUUACUUUUGGUACGCG (MIMAT0000137, SEQ ID10); mmu-miR-126-3p UCGUACCGUGAGUAAUAAUGC (MIMAT0000138, SEQ ID11); gga-miR-126 UCGUACCGUGAGUAAUAAUGCGC (MIMAT0001169, SEQ ID12); gga-miR-126* CAUUAUUACUUUUGGUACGCG (MIMAT0003723, SEQ ID13); rno-miR-126 UCGUACCGUGAGUAAUAAUGC (MIMAT0000832, SEQ ID14); rno-miR-126* CAUUAUUACUUUUGGUACGCG (MIMAT0000831, SEQ ID15): tni-miR-126 UCGUACCGUGAGUAAUAAUGC (MIMAT0002958, SEQ ID16); xtr-miR-126 UCGUACCGUGAGUAAUAAUGC (MIMAT0003588, SEQ ID17); and/or xtr-miR- 126* CAUUAUUACUUUUGGUACGCG (MIMAT0003587, SEQ ID19) or a complement thereof. In certain aspects, a subset of these miRNAs will be used that include some but not all of the listed miR-128 family members. In one aspect, miR-126 sequences have a consensus sequence of YAYYRUKASUWWURRUR (SEQ ID 3). In certain aspects, a subset of these miRNAs will be used that include some but not all of the listed miR-126 family members. In certain embodiments the mature sequences of miR-126 comprises all or part of hsa-miR-126 (MIMAT0000445, SEQ ID 1), or hsa-miR-126* (MIMAT0000444, SEQ ID 5).

A "miR-126 nucleic acid sequence" includes all or a segment of the full length precursor of miR-126 family members. Stem-loop sequences of miR-126 family members include hsa-mir-126 CGCUGGCGACGGGACAUUAUUACUUUUGGUACGCGCUGUGACACUUCAA ACUC GUACCGUGAGUAAUAAUGCGCCGUCCACGGCA (MI0000471, SEQ ID NO:4); bta- mir-126 UGACGGGACAUUAUUACUUUUGGUA CGCGCUGUGACACUUCAAACUCGUACCGUGAGUAAUAAUGCGCUGUCA (MI0004754, SEQ ID NO19 dre-mir-126 GAGCCAUUUUAACU GCUUCACAGUCCAUUAUUACUUUUGGUACGCGCUAGGCCAGACUCAAACU CGUACCGUGAGUAAUAAUGCACUGUGGCAGUGGGUUU (MI0001979, SEQ ID NO20:); fru-mir-126 CGGCCCAUUAUUACUUUUGGUACGCGC UAUGCCACUCUCAACUCGUACCGUGAGUAAUAAUGC(MI0003273, SEQ ID NO:21); gga-mir-126 GCUGGUGACGGCCCAUUAUUACUUUUGGUACG CGCUGUGACACUUCAAACUCGUACCGUGAGUAAUAAUGCGCUGUGGUCA GCA(MI000I244, SEQ ID NO22:); mmu- mir-126 UGACAGCACAUUAU UACUUUUGGUACGCGCUGUGACACUUCAAACUCGUACCGUGAGUAAUAAU GCGCGGUCA (MI0000153, SEQ ID 23:); rno-mir-126 UGACA GCACAUUAUUACUUUUGGUACGCGCUGUGACACUUCAAACUCGUACCGUG AGUAAUAAUGCGUGGUCA (MI0000898, SEQ ID 24); tni-mir-126 CGGCCC AUUAUUACUUUUGGUACGCGCUAUGCCACUCUCAACUCGUACCGUGAGUA AUAAUGC (MI0003274, SEQ ID 25); and/or xtr-mir-126 GGCUGUGCAUU AUUACUUUUGGUACGCGCUGUGUCACAUCAAACUCGUACCGUGAGUAAUA AUGCGCAG (MI0004827, SEQ ID 26) or a complement thereof.

The sequence of miR-126 has been highly conserved in evolution and is 100% conserved between humans, rats, dogs, chickens, zebrafish, and Fugu, as Illustrated in Wang et al., 2008, Dev. Cell, 15, p 261-271.

In some embodiments, the present invention is directed to miR-126 and to nucleotides having or comprising SEQ ID NO. 1 or SEQ ID NO. 2 and homologues thereof. In addition, the present invention is directed to fragments and variants of SEQ IDs: 1 and 2 that demonstrate the same effect as the nucleic acids of Seq. IDs.1 or 2. Accordingly, the present invention fragments and variants of SEQ ID NOs 1 and 2 having greater than about 70 %, or greater than about 75%, or greater than about 80 %, or greater than about 85 %, or greater than about 90 %, or greater than about 95 %, or greater than about 99 % of their nucleotides identical to those of SEQ IDs 1 or 2, and that bind to the miR-126 target gene(s) and modulate postnatal tissue repair. In addition, the present invention also provides fragments and variants of SEQ IDs 1 and 2 that differ from SEQ IDs 1 and 2 by a certain number of nucleotides. For example, in one embodiment, the present invention provides sequences that differ from SEQ ID NOs: 1 or 2 by no more than 10 nucleotides, or no more than 9 nucleotides, or no more than 8 nucleotides, or no more than 7 nucleotides, or no more than 6 nucleotides, or no more than 5 nucleotides, or no more than 4 nucleotides, or no more than 3 nucleotides, or no more than 2 nucleotides, or no more than 1 nucleotide, and that bind to the miR-126 target gene(s) and modulate postnatal tissue repair.

It is well known in the art that while in deoxyribonucleic acids the complementary nucleotide to Adenosine ("A") is thymidine ("T"), in ribonucleic acids the complementary nucleotide to A is uracil ("U"). Thus, the nucleotide T in a deoxyribonucleic acid is the equivalent of the nucleotide U in a ribonucleic acid, and vice versa.

For example, the nucleic acid-based miR-128 sequences of the present invention may comprise ribonucleotides, deoxyribonucleotides, 2'-modified nucleotides, phosphorothioate-linked deoxyribonucleotides, peptide nucleic acids (PNAs) , locked nucleic acids (LNAs), ethylene nucleic acids (ENA), certain nucleobase modifications such as 2-amino-A, 2-thio (e.g., 2-thio-U), G-clamp modifications, antagomirs, morpholinos, nucleic acid aptamers, or any other type of modified nucleotide or nucleotide derivative that is capable of Watson-Crick type base pairing with an miRNA. For example, in addition to naturally occurring DNA and/or RNA nucleotide bases, non-naturally occurring modified nucleotide bases that can be used in the miR-126 sequences of the invention include, but are not limited torn 8- oxo-guanine, 6-mercaptoguanine, 4-acetylcytidine, 5-(carboxyhydroxyethyl) uridine, 2'-O-methylcytidine, 5-carboxymethylamino -methyl-2-thioridine, 5-carb Ipseudouridine, beta-D-galactosylqueosine, 2'-Omethylguanosine, inosine, N. sup.6 - isopente nyladenosine, 1-methyladenosine, 1 -methylpseudouridine, 1-methylguanosine, 1-methylaminomethyllinosine, 2,2-dimethylguanosine, 2-methyladenosine, 2-methylguanosine, 3-methylcytidine, 5-methyleytidine, N,sup,6 - methyladenosine, 7-methylguanosine, 5-methylaminomethyluridine, 5- methoxyaminomethyl-2-thiouridine, beta-D-mannosylqueosine, 5-methoxycarbonylmethyluridine, 5-methoxyuridine, 2-methylthio-N6 isopentenyladenosine, N-((9-beta-D-ribofuranosyl-2-methylthiopurine-6-yl)carbamoyl)threonine, N-((9-beta-D -ribofuranosylpurine-6-yl) N-methylcarbamoyl) threonine, uridine-5-oxy acetic acid methylester uridine-5-oxyacetic acid, wybutoxosine, pseudouridine, queosine, 2-thiocytidine, 5 - methyl-2-thiouridine, 2- thiouridine, 2-thiouridine, 5-methyluridine, N-((9-beta-D -ribofuranosylpurine-6-yl) carbamoyl) threonine, 2'-O-methyl-5-methyluridine, 2'-0 -methyluridine, wybutosine, and 3-(3-amino-3-carboxypropyl) uridine.

For a review of some of the chemically modified nucleotides that can be used in the miR-126 sequences of the present invention, including 2'-O-methyl oligonucleotides, phosphorothioate-linked deoxyribonucleotides, morpholinos, and LNAs, see Esau (2008), vol. 44, p55-60; Summerton (2007), Current Topics in Medicinal Chemistry, vol. 7, p 651-660; and Krutzfeldt et al., 1005, Nature vol. 438, p685- 689, the contents of which are hereby incorporated by reference.

The miR-126 sequences of the present invention can also be attached to a peptide or a peptidomimetic ligand which may affect pharmacokinetic distribution of the miR-126 sequence such as by enhancing cellular recognition, absorption and/or cell permeation. The peptide or peptidomimetic moiety can be about 5-50 amino acids long, e.g., about 5, 10, 15, 20, 25, 30, 35, 40, 45, or 50 amino acids long. A cell permeation peptide can also include a nuclear localization signal (NLS). For example, a cell permeation peptide can be a bipartite amphipathic peptide, such as MPG, which is derived from the fusion peptide domain of HIV-I gp41 and the NLS of SV40 large T antigen (Simeoni et al, Nucl. Acids Res. 31 :2717-2724, 2003). Exemplary cell permeation peptides that may be conjugated to the miR-126 sequences of the present invention include Penetratin (RQIKIWFQNRRMKWKK, Seq. ID. 27), Tat fragment (GRKKRRQRRRPPQC, Seq. ID. 28), Signal sequence based peptide (GALFLGWLGAAGSTMGAWSQPKKKRKV, Seq. ID. 29), PVEC (LLIILRRRIRKQAHAHSK, Seq. ID. 30), Transportan (GWTLNS AGYLLKTNLKALAALAKKIL, Seq. ID. 31), Amphiphilic model peptide (KLALKLALKALKAALKLA, Seq. ID. 32), Arg9 (RRRRRRRRR, Seq. ID. 33), Cecropin PI (SWLSKTAKKLENSAKKRISEGIAIAIQGGPR, Seq. ID. 34), alpha.-defensin (ACYCRIPACIAGERRYGTCIYQGRLWAFCC, Seq. ID. 36), b-defensin (DHYNCVSSGGQCLYSACPIFTKIQGTCYRGKAKCCK, Seq. ID. 35), Bactenecin (RKCRIWIRVCR, Seq. ID. 37), PR-39 (RRRPRPPYLPR PRPPPFFPPRLPPRIPPGFPPRFPPRFPGKR-Nm, Seq. ID. 38) and Indolicidin ILPWKWPWWPWRR-NH2, Seq. ID. 39.

A peptide or peptidomimetic can be, for example, a cell permeation peptide, cationic peptide, amphipathic peptide, or hydrophobic peptide (e.g., consisting primarily of Tyr, Trp or Phe). The peptide moiety can be a dendrimer peptide, constrained peptide or crosslinked peptide. In another alternative, the peptide moiety can include a hydrophobic membrane translocation sequence (MTS). An exemplary hydrophobic MTS-containing peptide is RFGF having the amino acid sequence AAVALLPAVLLALLAP, Seq. ID. 40. An RFGF analogue (e.g., amino acid sequence AALLPVLLAAP, Seq. ID. 41) containing a hydrophobic MTS can also be a targeting moiety. The peptide moiety can be a "delivery" peptide, which can carry large polar molecules including peptides, oligonucleotides, and proteins across cell membranes. For example, sequences from the HIV Tat protein (GRKKRRQRRRPPQ, Seq. ID. 42) and the Drosophila Antennapedia protein (RQIKIWFQNRRMKWKK, Seq. ID. 43) have been found to be capable of functioning as delivery peptides. A peptide or peptidomimetic can be encoded by a random sequence of DNA, such as a peptide identified from a phage-display library, or one -bead-one-compound (OBOC) combinatorial library (Lam et al., Nature, 354:82- 84, 1991). The peptide or peptidomimetic which may be tethered to the miR-126 inhibitors of the invention may be a cell targeting peptide such as an arginine-glycine-aspartic acid (RGD)-peptide, or RGD mimic.

In other embodiments, the miR-126 inhibitors of the invention may be attached a cholesterol moiety, e.g., at the 3' or 5' end.

Accordingly, because the miR-126 containing nucleic acid molecules of the present invention may comprise or consist of either deoxyriboynucleotides or ribonucleotides, it is to be understood that every miR-126 sequence that is illustrated as comprising the deoxyribonucleotides A, C, T, and G, can equally comprise the ribonucleotides A, C, U, and G, where every position that is a T in the deoxyribonucleotide is substituted with a U in the ribonucleotide version, and vice versa.

In a preferred embodiment, the present invention relates to the isolated nucleic acid comprising a miR-126 nucleic acid sequence for use in the prophylaxis or treatment of atherosclerosis, its clinical manifestations and related disorders.

In particular, the miR-126 nucleic acid sequence can be used for the prophylaxis or treatment of tissue damages resulting in or due to coronary heart disease, like myocardial infarction, stroke, peripheral artery disease, aneurysm formation but also disease related to endothelial and vascular dysfunction such as pulmonary hypertension, transplant arteriopathy or various forms of vasculitis.

It has been found, that the use of miR-126 results in a decrease or attenuation of atherosclerosis. Moreover, it has been demonstrated that miR-126 is able to mobilize and recruit progenitor cells, e.g, SCA-1 + progenitor cells. In addition, the miR-126 nucleic acid sequences enable to stabilize vessel lesions.

Further, the nucleic acids of the present invention are particularly useful for use in tissue engineering, in particular in tissue engineering of blood vessels. For example, the miR-126 containing sequences allow mobilizing and recruiting progenitor cells as well as providing said progenitor cells for tissue engineering. In addition, the miR-126 containing sequences may be provided in form of vectors suitable for *in vivo* use namely suitable for transfection of target cells, thus, regulating CXCL12 to unleash an autoregulatory feedback loop.

The present inventors unravel a novel mechanism, by which AB derived from endothelial cells convey alarm signals to surviving cells in the vicinity to induce the chemokine CXCL12 which acts as a signal to mobilize progenitor cells and as an anti-apoptotic factor. The underlying process involves the release of an auto-regulatory, self-amplifying feedback loop, by which CXCL12 can trigger its transcription and secretion through its receptor CXCR4. This loop was set in motion by transfer of miR-126, which is enriched in endothelial AB and down-regulates the negative regulator RGS16 to unlock CXCR4 driving functional CXCL12 expression. Accumulation of specific miR in AB during endothelial apoptosis and namely miR-126-dependent silencing of signal elements after AB uptake enhance CXCR4 functions. Similarly, endothelial miR-128 has been found to govern vascular integrity and angiogenesis by regulating responses to VEGF. Targeted deletion of miR-126 in mice and its knock-down in zebrafish caused vascular leakage during embryonic development and impaired neovascularization after infarction, due to defects in endothelial cell proliferation, migration and angiogenesis. In a related mechanism, miR-126 enhanced the VEGF pathway by repressing SPRED1 as an intracellular inhibitor of angiogenic kinase signalling.

In the context of atherosclerosis, AB are released into the circulation, promote the mobilization and incorporation of Sca-1⁺ progenitor cells during plaque formation and limit atheroprogression in a CXCR4-dependent process. Further, AB carrying miR-126 or local transfer of miR-126 reduce macrophage and apoptotic cell content but increase CXCL12 expression and SMC content, overall resulting in smaller lesions with a less inflammatory phenotype. Notably, AB carrying miR-126 repress arterial Rgs16 expression, whereas AB deficient in miR-126 fail to reduce Rgs16 protein and to increase CXCL12 expression, establishing the mechanistic relevance of miR-126-mediated RGS16 repression *in vivo.*

In a preferred embodiment, the isolated nucleic acid comprising a miR-126 acid sequence allows to recruit Sca-1-positive progenitor cells, in particular CD31-positive progenitor cells of endothelial lineage or related to proangiogenic early-outgrowth cells (also known as endothelial progenitor or colony forming cells with phenotypic overlap to monocytes) to the site of tissue damage, thus, enabling tissue repair. In addition, the miR-126 nucleic acid sequence protects against atherosclerosis lesion formation as shown in the examples.

Particular preferred, the miR-126 are provided in form of apoptotic bodies. That is, the present invention provides in another embodiment apoptotic bodies containing nucleic acid molecules according to the present invention. These apoptotic bodies may be obtained by isolation from natural sources, i.e. from individuals or cell cultures. Alternatively, said apoptotic bodies/microparticles may represent synthetically produced apoptotic bodies containing the molecules sufficient to induce CXCL12 expression in target cells such as luminal endothelial cells, other vascular cells, such as pericytes or smooth muscle cells or immune cells.

The isolated nucleic acid molecules according to the present invention comprising the miR-126 nucleic acid sequence may also be provided in form of lipid membrane vesicles, like liposomes or other known means for transporting and targeting molecules to cells.

The nucleic acid according two the present invention as well as the apoptotic bodies or the pharmaceutical compositions containing said sequences are particularly preferred to be adapted for intravenous administration, or intraarterial administration, local drug-eluting preparations including stents and possibly for administration per inhalation (e.g. for pulmonary pathologies) or topical/subcutaneous administration (for skin affections).

In another embodiment of the present invention, pharmaceutical compositions containing said nucleic acids according to the present invention for use in the postnatal tissue repair or prophylaxis of tissue damages are provided. Said pharmaceutical compositions as well as the isolated nucleic acids according to the present invention are particularly useful for prophylaxis and/or treatment of postnatal tissue damage wherein the subject to be treated is a mammal, preferably a human.

The miR-126 sequences of the present invention may be incorporated into any pharmaceutical composition suitable for administration to the subject of choice and suitable for the chosen route of delivery. For example, the miR-126 sequences of the present invention can be formulated into a pharmaceutical composition comprising one or more pharmaceutically acceptable carriers. As used herein the term "pharmaceutically acceptable carrier" includes, but is not limited to, solvents, dispersion media, coatings, antibacterial and antifungal agents, isotonic and absorption delaying agents, and the like, compatible with pharmaceutical administration. The use of such pharmaceutical carriers is well known in the art (see, for example, Remington's Pharmaceutical Sciences, 17th Edition, 1985, Publisher: Mack Pub. Co., the contents of which are hereby incorporated by reference).

The exact formulation chosen for the pharmaceutical compositions of the invention will vary depending on the intended delivery route and the desired release profile. For example, formulations suitable for direct injection and parenteral administration may include sterile aqueous solutions which may also contain buffers, diluents, preservative, and other suitable additives, and for intravenous use, the total concentration of solutes may be controlled to render the preparation isotonic. Formulations suitable for sustained release may also be produced, for example where preventative or long-term treatments are desired. One of skill in the art can readily formulate the miR-126 sequences of the invention into a pharmaceutical composition suitable for the chosen delivery route and chosen release profile without undue experimentation.

### Administration and Delivery

The miR-126 sequences of the present invention, whether to be administered as isolated oligonucleotides or to be delivered using an expression vector or transporting molecules, like lipid membrane vesikel, e.g. liposoms etc. , can be delivered to a subject by a variety of routes, including, but not limited to oral delivery, parenteral administration, intravenous or intraarterial delivery, intramuscular, subcutaneous delivery, intraperitoneal, intrathecal or intraventricular, delivery by inhalation, oral intrathecal, parenchymal, nasal, oral, intraocular delivery, topical delivery, (including ophthalmic, intranasal, and transdermal delivery) and the elution from or coating of devices, e.g. intravascular stents.

The miR-126 sequences of the invention can also be administered in a single dose or in multiple doses, as desired, and can be administered in combination with other pharmaceutical agents or treatment regimens if desired, for example, in conjunction with other therapeutic agents designed to allow postnatal tissue repair. For example, the miR-126 sequences of the present invention can be administered in conjunction with another agent or treatment regimen useful for treating atherosclerosis.

In preferred embodiments, the miR-126 sequences of the present invention are delivered directly to the cells or tissue in which they are needed, for example the tissue in which it is desired to enhance tissue repair. For example, the miR-126 sequences of the invention can be delivered directly to the blood vessels to be targeted, or can be conjugated to a molecule that targets the miR-126 sequences to those areas.

In one embodiment, the miR-126 sequences of the invention may be administered to cells ex-planted from a subject followed by reintroduction of the cells into the subject, i.e. by ex vivo delivery. Such methods may be particularly desirable in the case of delivery of an expression vector but can also be used for delivery of oligonucleotides. Such ex vivo methods are known in the art. Suitable methods that could be used include virtually any method by which a nucleic acid may be delivered to a cell, including, but not limited to, transfection, by injection including microinjection by calcium phosphate precipitation by using DEAE-Dextran followed by polyethylene glycol by direct sonic loading by liposome - mediated transfection and receptor-mediated transfection by microprojectile bombardment by agitation with silicon carbide fibers by desiccation/inhibition-mediated DNA uptake and/or any combination of such methods. Some of the above methods may also be used to deliver the miR-126 sequences of the invention directly to the subject, i.e. without first removing the cells and supplying the miR-126 sequences to the cells ex vivo.

### Doses & Effective Amounts

The miR-126 sequences of the invention should be administered to a subject in an amount that is effective for the desired purpose, for example in an amount that is effective to allow tissue repair or protection, in particular to prevent or treat atherosclerosis. One of skill in the art can readily determine a suitable dose for administration by taking into account factors such as the size and weight of the subject; the extent of the neovascularization or disease penetration; the age, health and sex of the subject; the route of administration; and whether the administration is regional or systemic, and by using performing tests using in vitro models or in vivo models using test animals and the like.

Further, the present invention provides methods for the treatment of individuals for postnatal tissue repair or for the prophylaxis of tissue damage. In particular, the present invention provides methods for the prophylaxis or treatment of atherosclerosis including the step of providing isolated nucleic acid molecules according to the present invention comprising a miR-126 nucleic acid sequence. For example, the method according to the present invention are useful for the prophylaxis or treatment of tissue damage resulting in or due to coronary heart diseases, heart attack, stroke or aterial obstructive disease.

The present invention will be described in more detail with the help of examples below. However, the examples are not intended to restrict the claimed matter.

### Example

### Material and Methods

### Cell culture, AB collection and pretreatments

Human aortic smooth muscle cells (SMCs, PromoCell) were cultured in SMC-2 growth medium (PromoCell) as described, Krohn, R., et al. Circulation 116, 1812-1820 (2007). Human umbilical vein endothelial cells (HUVECs) and aortic endothelial cells (ECs) isolated from Apoe-/- mice, Zernecke, A., et al. Circulation 113, 2120-2127 (2006), were cultured in EC growth medium (PromoCell) and used between passage 4-5. Apoptotic bodies (AB) were isolated from supernatants of apoptotic HUVECs after serum- and growth factor-starvation for 24 h by sequential centrifugation steps, Hristov, M., Erl, W., Linder, S. & Weber, P.C. Blood 104, 2761-2766 (2004). For the collection of AB after transfection of HUVECs, apoptosis was induced after 4 hours. AB were treated with DNase (Qiagen), RNase (Fermentas) and proteinase K (Sigma) for 30 min after sonication for 20 s. Lipids were extracted from AB according to Bligh and Dyer with two sequential extraction steps in methanol/chloroform and lipids were collected after centrifugation (4000 g, 10 min, 10°C) from the lower chloroform phase after evaporation with N2. HUVECs (5x10⁵) were treated with AB derived from 2x10⁶ apoptotic HUVECs or CXCL12 (100 ng/mL, R&D Systems) or AMD3100 (1 µg/mL), Zernecke, A., et al. Circ. Res. 102, 209-217 (2008), for 6 h unless indicated otherwise. AB were also collected from human atherosclerotic plaque material, Leroyer, A.S., et al. J. Am. Coll. Cardiol. 49, 772-777 (2007) and mouse whole blood by FACS sorting. Human carotid artery endarterectomy specimens were rinsed in sterile PBS (with 100 U/mL streptomycin/penicillin), minced using fine scissors and vigorously resuspended. After centrifgation (800 g, 10 min, 20°C) to remove debris, AB were pelleted (16000 g, 20 min, 4°C) for antibody staining. EDTA-buffered whole blood obtained from miR-126+/+ wild-type or mi-R126-/- mice, Wang, S., *et al*, supra was centrifugated (800 g, 10 min, 20°C) to obtain AB-rich plasma for antibody staining. Samples were diluted in staining buffer (BD Bioscience) and incubated with FITC-conjugated annexin-V (BD Biosciences) and with APC-conjugated anti-human CD31 (eBioscience) or PE-Cy7-conjugated anti-mouse CD31 (Abcam) antibodies. Annexin-V+ CD31+ AB were sorted using a FACSAria and FACSDiva software (BD Biosciences).

### Chip array

Total RNA was isolated from AB or HUVECs and microRNA (miR) was purified using the mirVanaTM miRNA Isolation Kit (Ambion). miR obtained from 10 µg of total RNA was labeled using the mirVanaTM miRNA Labeling Kit (Ambion) and fluorescent Cy3 (Molecular Probes), and hybridized to the Ambion mirVanaTM miRNA Bioarray (1566 v.1, www.ambion.com/techlib/prot). Hybridized mirVana miRNA Bioarrays were scanned and quantified by using imaGene 5.5.4 (Bio Discovery). Microarray data have been uploaded to the GEO database (accession: http://www.ncbi.nim.nih.gov/geo/query/ acc.cgi?token= rhwdvoikkasaqro&acc=GSE12696). Resulting signal intensities were background-corrected and then normalized using variance stabilization (VSN package, Bioconductor project). To calculate the relative miRNA expression levels in AB, an average baseline was calculated using values of the empty spots. Sample spots with 4-fold or higher intensities over baseline (log2≥2) were selected for illustration in the graph. Ratios of miR enriched in AB with 2-fold intensity (log2≥1) compared to HUVECs are depicted in the right column.

### RT-PCR and real time-PCR

The expression of specific miR in AB was analyzed by quantitative miR stem loop RT-PCR technology (Ambion). Highly target-specific stem loop structure and reverse-transcription primer sets (miR-126, AM30023, miR-let7i, AM30004, miR-let7c, AM30002 and U6, AM30303, all Ambion) were used for miR amplification, allowing only mature miR targets to form reverse transcription primer/mature miR chimeras extending the miR 5'-end. Isolated RNA (Qiagen) was reverse-transcribed into cDNA by Mo-MLV (Invitrogen). PCR was performed using 20 ng cDNA, Taq polymerase (Promega) and specific primers (aldolase: 5'-AGCTGTCTGACATCGCTCACG-3', Seq. ID No. 44, 5'-CACATACTGGCAGCGCTTCAAG-3', Seq. ID 45; CXCL12 5'-CGCCACTG CCTTCACCTCCTC-3', Seq. ID 46, 5'-GGCATACATAGGCTTCAGAGGCAATC. 3', Seq. ID 47). Products were separated by agarose gel electrophoresis. Real-time PCR was performed using the QuantiTect SYBR-Green PCR-kit (Opticon MJ Research) or Taqman Gene expression master mix (Applied Biosystems) and specific primer pairs (18S rRNA, Hs03003631_g1, 4333760F; CXCL12, Hs00171022_m1 and Mm00445552-m1, all Applied Biosystems, and RGS16, 5'-AGGGCACACCAGATCTTTGA-3', Seq. ID. 48, 5'-GTCTGCAGGTTCATCC TCGT-3', Seq. ID. 49).

### HUVEC transfections and luciferase assays

Transfection of HUVECs was performed using the HUVEC Nucleofector Kit (Amaxa) according to the manufacturer's protocol. To investigate functional RNA transfer by AB, HUVECs (2.10 ⁶) were transfected with silencer EGFP siRNA (1.5 pg, AM4626, Ambion). After 6 h of recovery, apoptosis was induced by serum starvation, AB were isolated and added to HUVECs (0.5-1 10⁵) transfected with pEGFP (3 µg, Clontech). Gain- and loss-of-function assays were performed in HUVECs (0.5-1 10⁶) transfected with 200 pmol (~2.5 µg) pro-miR-126, anti-miR-126, let-7c or scrambled control (Ambion), RGS18 siRNA, SPRED1 siRNA, or scrambled control siRNA (Santa Cruz) or with 2 µg of pCMV-scrambled or pCMV-RGS16 after 48-72 h of recovery. RGS16 cDNA was amplified from total RNA of HUVECs and cloned into a pCMV vector using Kpnl and Sacl restriction sites (Clontech). To study CXCL12 expression in HUVECs, HUVECs (5x10⁵) were transfected with a plasmid carrying the luciferase reporter gene (pGL-4-SDF) under the control of the CXCL12-promoter, Garcia-Morujo, C., et al. J. Mol. Biol. 348, 43-62 (2005), or mock-pGL-4 (5 µg) and luciferase activity was determined after 12 h using Bright-Glo luciferase kit (Promega). Predicted binding sequences for miR-126 were identified using http://microrna.sanger.ac.uk in the 3' untranslated region (3'UTR) of the following target genes, e.g. SPRED1 sense: 5'-TTTAACTAAAT'GTAAGGTACGA-3', Seq. ID. 50, VCAM-1 sense: 5'-TGTATA GTACTGGCATGGTACGG.3', Seq. ID. 51; RGS16 sense: 5'-GCCAGTGTTT TTTGTGOTATGA.3', Seq. ID. 52; RGS16 mutated/ antisense: 5'-GCGTGA TAATGTGACCATGCT-3', Seq. ID. 53; CXCL12 sense: 5'-TGCATTTATAG CATACGGTATGA-3', Seq. ID. 54, CXCL12 mutated/ antisense: 5'-GCGTT AATAAGGATTGCCA TTT-3', Seq, ID. 55. These sequences were inserted into the multiple cloning site of the pMIR-Report vector (Ambion), thus located in the 3'UTR downstream of the luciferase reporter cDNA. All vectors (3 µg each) were co-transfected with pro-miR-126 precursor oligonucleotide (200 pmol) into HUVECs (5x10⁵) and the luciferase activity detected using the Bright-Glo luciferase kit (Promega) after 48 h.

### Flow cytometry

HUVECs were trypsinized, fixed (Cytofix, BD Biosciences), permeabilized with methanol and stained using an ERK1/2 antibody (Cell Signaling) detected by FITC-conjugated secondary antibody (Sigma), Alexa-Fluor 647-conjugated PERK1/2 antibody (Cell Signaling) or corresponding isotype control antibodies (Cell Signaling) according to the manufacturer's protocol. Whole blood obtained from the retro-orbital plexus of mice was EDTA-buffered, subjected to red-cell lysis (Pharmlyse BD Biosciences) and washed in DMEM with 2 mM EDTA and 0.5% bovine serum albumin (BSA). Blood leukocytes were reacted with combinations of specific antibodies to Gr-1, CD11b, CD3, CD19, CD45 (BD Biosciences), antibodies to CD115, CD4, CD8 (eBiosciences), with a mouse anti-lineage biotin-antibody cocktail (Mittenyi) detected by a FITC-conjugated secondary antibody and with a PE-conjugated antibody to Sca-1 (BD Biosciences) in HBSS with 0.3 mM EDTA and 0.1% BSA. To analyze the content of circulating annexin-V+CD31+CD41. AB, 100 µL of EDTA-buffered whole blood was centrifuged (800 g, 10 min) to obtain AB- rich plasma and stained with PE-Cy7-conjugated antibodies to CD31, FITC-conjugated annexin-V and PE-conjugated CD41. Apoptosis of HUVECs was quantified using the annexin-V-FITC Apoptosis Detection Kit I (BD Biosciences). Samples were analyzed after appropriate fluorescence compensation and gating strategies using a FACSCanto-II or a FACSAria with FACSDiva (BD Biosciences) and FlowJo software (Treestar).

### Western blotting and ELISA

HUVECs were lysed in Cyto buster (Novagen) or 20 mM Tris-Cl pH 8.0, 2 mM EDTA, 150 mM NaCl, 1% NP-40, supplemented with 1x complete protease inhibitor cocktail (Roche). To analyze phosphorylated proteins, HUVECs were lysed in 1x PBS, pH 7.6, 1% Triton-X100, 2.5 mM EDTA, 2.5 mM EGTA, supplemented with phosphatase inhibitors (100 mM NaF, 5 mM Na₃VO₄, 30 mM Na₄P₂O₇) and 1x complete protease inhibitor cocktail. Insoluble material was removed by centrifugation (18,000 g, 5 min, 4°C). Total protein was resuspended in 2x Laemmli loading buffer, boiled for 10 min supplemented with ß-mercaptoethanol, resolved by 12% SDS-PAGE and transferred to PVDF membranes. Proteins were detected with antibodies to RGS3 (Santa Cruz), RGS16 (Abcam), P-ERK1/2, ERK1/2 (Cell Signaling) and ß-actin (Sigma), using HRP-conjugated secondary antibodies (Pierce, Santa Cruz) and enhanced chemiluminescence (GE Healthcare). CXCL12 concentrations in HUVEC lysates and supernatants were quantified using the Duo-Set ELISA Development Kit (R&D Systems).

### Mouse models of atherosclerosis

*Apoe*^{*-*/*-*} mice (C57BL/6 background) were i.v. injected with AB obtained from 1x10⁸ apoptotic HUVECs or PBS (control) and blood was collected from the retro-orbital plexus after 24 h. *Apoe*^{*-*/*-*} mice were fed a high-fat diet (21% fat, 0.15% cholesterol, Altromin) for 6 weeks for the assessment of early diet-induced atherosclerosis, Zernecke., et al., 2008, supra. These mice were injected twice per week intravenously with PBS (200 µl) or AB from 1x10⁸ apoptotic HUVECs (in 200 µL PBS). Some *Apoe*^{*-*/-} mice were continuously treated with AMD3100 (Sigma, 120 µg/d) administered via osmotic mini pumps (Alzet, model 2000), and some mice were injected once per week with 3x10⁵ AB sorted from human endarterectomy plaque material or PBS for 6 weeks. Bilateral perivascular non-constrictive silastic collars were placed around carotid arteries in *Apoe*^{*-*/-} mice that had received a high-fat diet for 2 weeks immediately followed by the application of pro-miR-126, let-7c or scrambled controls (10 µg/carotid artery) mixed with pluronic gel (25% w/v, Pluronic F127, Sigma), Bot et al. The intraluminal incubation of common carotid arteries of *Apoe*^{-/-} mice fed a high fat diet for 10 weeks was performed by injecting 10⁴ AB collected from wild-type or miR126^{-/-} mice (in 100 µL PBS) via a flexible tubing (Cornstar) inserted into the external carotid artery with the proximal common carotid artery and the distal internal and external branches closed with sutures. After 30 min, the AB suspension was retrieved, closed arteries were flushed once with PBS and the blood flow was reconstituted in the common carotid artery via the internal carotid artery. Mice were sacrificed after 5 more weeks of diet. Animal experiments were approved by local authorities and complied with German animal protection law.

### Morphometry and Immunohistochemistry

Hearts, aortas and carotid arteries were harvested by *in situ* perfusion fixation with 4% paraformaldehyde, 20 mM EDTA and 5% sucrose. The extent of atherosclerosis was assessed in aortic roots by staining for lipid deposition with oil-red-O, Zernecke et al., 2008, supra in 5-µm transversal sections. After paraffin embedding, neointimal and medial areas were quantified in Movat's pentachrome-stained serial 5-µm sections of the right common carotid artery within a standardized distance (100 µm) distal and proximal to the maximal, collar-induced plaque volume. Immunofluorescence staining was performed using antibodies to MOMA-2 (MCA519, Serotec), CD68 (EPR1392Y, Epitomics, California), α-smooth-muscle actin (1A4), CD3epsilon(Abcam, Cambridge), anti-CD31 (M-20, Santa-Cruz Biotechnology) and Sca-1 (Ly-6A/E, E13-161.7, BD Biosciences) or appropriate isotype controls (Santa Cruz or BD Biosciences) visualized by FITC- or Cy3-conjugated-secondary antibody (Jackson ImmunoResearch). Apoptotic nuclei were detected by terminal deoxynucleotidyl nick-end labeling (TUNEL in situ cell death detection kit, Roche). Collagen was stained using Sirius red (Polysciences). TUNEL⁺ cells were quantified as positive nuclei relative to total cell nuclei in the aortic root. Nuclei were counter-stained by 4',6-Diamidino-2-phenylindol (DAPI). Images were recorded with a Leica DMLB fluorescence microscope and CCD camera and image analysis was performed using Diskus Software (Hilgers, Königswinter).

### AnnexIn-V-FITC gold labelling and scanning electron microscopy

Untreated or serum-starved HUVECs were fixed with 1% glutaraldehyde and 2% paraformaldehyde. Unreacted aldehydes were quenched with 0.1 M glycine. Fixed cells were stained with annexin-V-FITC followed by incubation with anti-FITC antibody conjugated to gold particles (10 nm, Ted Pella). After postfixation with 1% glutaraldehyde, dehydrated samples were processed by critical point drying with CO₂ and sputter-coated with carbon. Scanning electron microscopy images (FEI/Philips ESEM XL30 FEG) were acquired in secondary electron detection, back-scattered electron detection, or mixed detection mode.

### Immunofluorescence

HUVECs were grown on glass chamber slides (Nunc). AB were stained with annexin-V-FITC (BD Biosciences) for 30 min and incubated with sub-confluent HUVECs for 1 h. After washing and fixation/permeabilization, filamentous actin (F-actin) of adherent cells was visualized using Alexa-568 phalloidin (Invitrogen) as described, Hristov, et al., supra.

### Statistical Analysis

Data represent mean±s.e.m. and were compared by one-tailed Student's t-test, ANOVA or non-parametric Mann-Whitney test or Kruskal-Wallis test with Dunn's post-hoc test (InStat software, GraphPad) as appropriate. The null hypothesis was rejected at the 0.05 level.

### Example 1

### Endothelial AB confer RNA to induce CXCL12

As known in the art, human umbilical vein endothelial cells (HUVECs) took up annexin-V-Iabeled AB derived from serum-starved apoptotic HUVECs, involving a re-arrangement of cytoskeletal fibres, as visualized after 2 h by confocal microscopy and co-staining for F-actin **(****Fig. 1A****)**. In response to endothelial AB, *CXCL12* transcription was up-regulated, as evident by real-time RT-PCR in HUVECs, SMCs or mouse aortic ECs or in HUVECs by *CXCL12* luciferase-reporter assay **(****Fig. 1B****,C).** This induction was time-dependent, observed as early as after 6 h **(****Fig. 1D****),** and entailed a marked increase in intracellular and secreted CXCL12 protein compared with vehicle-treated HUVECs, as determined by flow cytometry (data not shown) or ELISA **(****Fig. 1E****)**.

Cells undergoing apoptosis can secrete chemoattractants, eg phospholipids, as eat-me signals for phagocytes. The up-regulation of *CXCL12* mRNA was seen with sonicated AB but not after treatment with AB-conditioned supernatants, indicating that AB do not secrete but contain crucial mediators **(****Fig. 1F****)**. To identify the constituents required for CXCL12 induction, a sub-characterization of AB has been performed. Treatment with lipid-extracts of AB did not up-regulate *CXCL12* expression, nor did pre-treatment of AB with DNase or proteinase K interfere with *CXCL12* induction, excluding that lipids, DNA or protein components account for the effects **(****Fig. 1F****).** In contrast, AB pretreated with RNase failed to up-regulate *CXCL12* transcript expression **(****Fig.1F****)**, indicating a RNA component as the responsible mediator. Substantial levels of *CXCL12* transcripts were detectable in HUVECs and apoptotic HUVECs but not in AB, revealing that *CXCL12* mRNA is not directly conferred to recipient cells but rather induced by AB **(****Fig. 1F****),**

Horizontal transfer of whole genes and mRNA through exosomes or progenitor cell-derived microvesicles can be functionally operative, *eg* in activating angiogenic programs in endothelial cells, as described. It has been verified by flow cytometry that FITC-labeled oligonucleotides can be conferred to HUVECs by incubation with AB derived from FITC⁺-oligo-22-mer-transfected HUVECs **(****Fig. 1G****)**. Moreover, treatment with AB collected from HUVECs transfected with a pEGFP-silencing siRNA dose-dependently reduced the fluorescence of HUVECs transfected with a pEGFP reporter vector **(****Fig. 1H****)**, corroborating a functional RNA transfer by AB to recipient cells,

### Example 2

### MIR-126 In endothelial AB mediates CXCL12 Induction

MicroRNAs (miR) constitute a class of highly conserved non-coding RNAs, which control gene expression by posttranscriptional destabilization and translational inhibition of mRNAs, and participate in the regulation of diverse cellular processes. To probe whether AB contain mIR that account for the up-regulation of CXCL12 after Intercellular transfer into endothelial cells, the prevalence of miR in HUVEC-derived AB was analyzed by chip arrays, revealing a distinct expression profile for several miR **(****Fig. 2A****).** Notably, miR-126 was most highly expressed in AB, relative to other miR detected by chip array, as confirmed by real time-PCR **(****Fig. 2A,B**). Further to studies demonstrating miR-126 expression in HUVECs, the present data unveil that miR-126 was among the few miR enriched in AB (log₂ ratio >1) as compared to untreated HUVECs **(****Fig. 2A****),** thus the only, miR highly expressed and enriched in AB. In contrast, let-7c was highly expressed in AB but not enriched in AB over HUVECs **(****Fig. 2A****)**. Notably, treatment with endothelial AB markedly increased relative miR-126 expression levels in HUVECs **(****Fig. 2C****)**, confirming its transfer to recipient cells.

Surveying bioinformatic databases (*e.g*. miRanda) based on thermodynamic binding efficiency identified CXCL12, vascular cell adhesion molecule-1 (VCAM-1) and members of the regulator of G-protein signaling (RGS) family, including RGS3 and RGS16, as possible molecular targets of miR-126. Human and mouse miR-126 are identical and have complementary binding sites in RGS16 and Rgs16. Besides VCAM-1, sprouty-related protein 1 (SPRED1) has been confirmed as a target of miR-126. To scrutinize whether miR-126 controls the expression of these targets, we cloned their predicted miR-126-binding sites (or mutated control sequences) have been cloned in the 3'UTR downstream of a luciferase gene in the pMIR-Report plasmid. Co-transfection of pro-miR-126 with these vectors in HUVECs repressed luciferase expression through the miR-126-binding sites of *SPRED1, VCAM-1, RGS16* and *CXCL12* **(****Fig. 2****D),** indicating these genes to be targets of miR-126. Mutated controls had no effect (data not shown). However, HUVECs transfected with pro-miR-126 but not with anti-miR-126, scrambled control or let-7c (as an additional control miR highly expressed in AB) displayed an up-regulation of *CXCL12* mRNA and CXCL12 protein expression in supernatants and lysates **(****Fig. 2****E,F.** data not shown).

### Example 3

### MIR-126 targets RGS16 to unleash CXCL12 induction via CXCR4

The data as described herein indicate that targets of miR-126 other than *CXCL12* itself *e.g. RGS16,* may be more relevant for *CXCL12* expression. This may be due to higher and less susceptible transcript expression of *CXCL12* 20-fold exceeding that of *RGS16,* as evidenced by real-time PCR (not shown). Accordingly, SMC-derived AB, which only contained trace amounts of miR-126 induced a more moderate up-regulation of *CXCL12* mRNA in HUVECs than endothelial AB **(****Fig. 1B****)**, which may be mediated by alternative signals or mechanisms unrelated to the transfer of endothelial-specific miR-126, as In SMCs. Moreover, RGS16, a negative regulator of the CXCL12 receptor CXCR4, was considerably higher expressed in HUVECs than RGS3, suggesting that RGS16 is the predominantly expressed RGS member in HUVECs, whereas *e.g*. Jurkat T cells expressed RGS3 and RGS16 at almost equivalent levels. As analyzed by Western blotting, RGS16 expression in HUVECs was reduced by transfection with pro-miR-126 but not with anti-miR-126, scrambled control or let-7c, and by treatment with AB, but increased by treatment with AB from anti-miR-126-transfected HUVECs **(****Fig. 2G****)**. Posttranscriptional stability of *RGS16* mRNA was unaffected (data not shown), indicating that miR-126 silenced RGS16 translation. Of note, siRNA-mediated knock-down of *RGS16* but not of *SPRED1* enhanced *CXCL12* mRNA expression **(****Fig. 2H****).** Overexpression of RGS16 in HUVECs almost completely abolished *CXCL12* mRNA levels, indicating that it acts as a strong suppressor of CXCL12 expression **(****Fig. 2H**). These data imply that RGS16 may control CXCL12 homeostasis through CXCR4. Indeed, overexpressing RGS16 or blocking CXCR4 with the small-molecule antagonist AMD3100 inhibited the up-regulation of *CXCL12* expression by AB or pro-miR-126 in HUVECs, corroborating an involvement of REGS16-restricted CXCR4-mediated signals in CXCL12 regulation **(****Fig. 2I****)**. Notably, AB derived from anti-miR-126-transfected but not mock-transfected HUVECs failed to induce but rather reduced CXCL12 expression compared to controls, pinpointing the transfer of miR-126 to underlie the effects of AB **(****Fig. 2I****)**. Interestingly, CXCL12 itself induced an up-regulation of *CXCL12* mRNA in HUVECs through CXCR4, as shown by inhibition with AMD3100 **(****Fig. 2I****)**, thus establishing an auto-regulatory feed-back loop. This is supported by findings that CXCL12-induced ERK1/2 phosphorylation mediated via CXCR4 (and inhibited by AMD3100) is amplified by AB or pro-miR-126 and by silencing *RGS16* but not *SPRED1* in HUVECs, as evident by Western blotting **(****Fig. 2****J)** or flow cytometry. Reciprocal effects of RGS16 and SPRED1 on ERK activity induced by CXCL12 and VEGF may be due to differences in signal pathways of Gₗ-coupled and angiogenic receptors. These data suggest that RGS16 to be the mechanistically relevant target of miR-126.

### Example 4

### Endothelial AB Induce Cxcl12 and progenitor recruitment In atherosclerosis

Low CXCL12 plasma levels have been clinically associated with unstable coronary artery disease, suggesting anti-inflammatory or plaque-stabilizing properties of CXCL12 in human atherosclerosis. Indeed, the CXCL12/CXCR4 axis has been shown to exert direct atheroprotective effects in apolipoprotein E-deficient (*Apoe*^{-/-}) mice by controlling myeloid cell homeostasis. Endothelial damage is thought to precede the development of atherosclerotic lesions at predilection sites and is associated with formation and release of annexin-V⁺ AB. Indeed, increased circulating apoptotic microparticles correlate with impaired endothelial function in coronary artery disease, as described before. Feeding *Apoe*^{-/-} mice a high-fat diet increased the levels of circulating annexin-V⁺CD31⁺ AB at 6 weeks (2.0±0.9-fold increase, data not shown) and even further at 30 weeks compared to normal chow **(****Fig. 3A****)**. Given the link between endothelial AB and induction of bone-marrow cell-mobilizing CXCL12, we tested whether intravenous injection of AB mobilizes progenitor cells to peripheral blood. Flow cytometry revealed that circulating Sca-1⁺ lineage⁻ progenitor cells increased in *Apoe*^{-/-} mice 1 day after AB injection **(****Fig. 3B****)**, Peripheral blood monocyte/neutrophil counts were unaltered (data not shown), indicating a specificity of AB for mobilizing progenitor cells while pacifying the myeloid compartment.

Repetitive injections of in *Apoe*^{*-*/-} mice twice weekly during a high-fat diet feeding for 6 weeks increased the luminal incorporation of Sca-1⁺ cells into aortic root plaques in a CXCR4-dependent process, as apparent by inhibition with continuous application of AMD3100 via osmotic mini-pumps **(****Fig. 3C****)**. Double-immunofluorescence staining revealed a co-localization of Sca-1 with luminal CD31⁺ endothelial cells **(****Fig. 3D****)** and few CD68⁺ macrophages but not with alpha-smooth-muscle actin⁺ SMCs or CD3⁺ T cells, suggesting that AB primarily recruit CD31⁺ endothelial progenitor cells for repair during early plaque growth. Accordingly, chronic AB treatment induced the expression of Cxcl12 foremost in luminal cells of aortic root plaques after 6 weeks of high-fat diet in *Apoe*^{*-*/*-*} mice **(****Fig. 3E****)**.

### Example 6

### Endothelial AB protect against atherosclerosis

Prolonged treatment with AB reduced atherosclerotic lesion formation and plaque size in the aortic root of *Apoe*^{*-*/*-*} mice associated with a decreased macrophage and apoptotic cell content in comparison to control-treated mice, which was reversed by application of AMD3100 **(****Fig. 4A****B**). To verify that the anti-atherogenic mechanism exerted by HUVEC-derived AB can be translated into similar effects by AB generated in atherosclerotic mice or patients, endothelial AB carrying miR-126 have been isolated from atherosclerotic plaques of patients undergoing endarterectomy of the carotid arteries. Remarkably, systemic treatment of *Apoe*^{-/-} mice on a high-fat diet for 6 weeks with atherosclerotic plaque-derived AB resulted In a reduction of plaque area and macrophage content in the aortic root **(****Fig. 4C, D****)**, indicating that endothelial AB in general serve as a compensatory signal to confer atheroprotection.

Collectively, the data described herein support the notion that AB released by apoptotic endothelial cells may function as a paracrine alarm system to mobilize progenitor cells for endothelial repair, evoking a protective response in diet-induced atherosclerosis, which may be related to the induction of CXCL12 as an anti-apoptotic survival factor. Like CXCL12, treatment with AB was found to protect HUVECs from apoptosis and induced their proliferation, paralleling findings in endothelial progenitor cells treated with HUVEC-derived apoptotic bodies. In contrast, no effects on apoptosis or proliferation of SMCs or Jurkat T cells treated with AB have been observed, suggesting a cell type-specific susceptibility to endothelial AB or AB-induced CXCL12 with a predominant responsiveness of endothelial cells, which may be related to the endothelial origin of miR-126. In addition, miR-126 has been found to inhibit VCAM-1 expression in endothelial cells, thereby limiting inflammatory cell recruitment, This may contribute to reduced monocyte influx in AB-treated *Apoe*^{*-*/*-*} mice, explaining the partial modulation of the plaque phenotype by CXCR4 blockade.

### Example 6

### MIR-126 mediates atherorpotective effects of endothelial AB

To scrutinize the local effects of AB-conferred miR-128 on the extent and quality of advanced atherosclerosis, a model of collar-induced accelerated plaque formation mediated by proximal flow reduction, Bot, I., et al. Circulation 115, 2516-2525 (2007)., has been employed. Carotid arteries of *Apoe*^{*-*/*-*} mice were transduced with miR-128, scrambled control or let-7c embedded in pluronic gel directly after collar placement. Carotid arteries treated with miR-126 but not let-7c displayed a marked reduction in plaque area and an up-regulation of *Cxcl12* expression **(****Fig. 5A,B****)**. As In mice chronically treated with AB, this was associated with reduced content of macrophages and apoptotic cells in miR-126-treated arteries, as well as with an increased relative content of intimal SMCs **(****Fig. 5C****),** which may be attributable to a CXCL12-mediated recruitment of progenitor calls. In addition, collagen content, as evident by Sirius-red staining, was higher in miR-126-treated than in scrambled control-treated arteries **(****Fig. 5D****),** consistent with a more stable plaque phenotype. To study the effects of local AB-mediated delivery of miR-126 in the context of diet-induced atherosclerosis, a model for local intraluminal exposure of carotid arteries of *Apoe*^{-/-} mice to endothelial AB isolated from *miR-126*^{*+*/*+*} or *miR-126*^{*-*/*-*} mice has been developed. The results clearly indicate that miR-126-carrying but not mIR-126-deficient AB conferred a protection against diet-induced atherosclerosis in the carotid artery **(****Fig. 5E****),** which was associated reduced macrophage infiltration and increased SMC content **(****Fig. 5G****).** Notably, a marked induction of *Cxc*/*12* mRNA expression in carotid arteries incubated locally with miR-126-carrying but not miR-126-deficient mouse AB was accompanied by a reduction in Rgs16 protein expression **(****Fig. 5F****)**. Collectively, these data clearly establish that the atheroprotective effects of AB are mediated by miR-126 targeting RGS16.

### Conclusion

The experiments described herein unravel a novel mechanism, by which AB derived from endothelial cells convey alarm signals to surviving cells in the vicinity to induce the chemokine CXCL12 as a crucial signal for the mobilization of progenitor cells and as an anti-apoptotic factor. The underlying process involves the release of an auto-regulatory, self-amplifying feedback loop, by which CXCL12 can trigger its transcription and secretion through its receptor CXCR4. This loop is unleashed by transfer of miR-126, which is enriched in endothelial AB and down-regulates the negative regulator RGS16 to unlock CXCR4 driving functional CXCL12 expression. Accumulation of specific miR in AB during endothelial apoptosis and namely miR-126-dependent silencing of signal elements after AB uptake enhance CXCR4 functions. Similarly, endothelial miR-126 has been found to govern vascular integrity and angiogenesis by regulating responses to VEGF. Targeted deletion of miR-128 in mice and its knock-down in zebrafish caused vascular leakage during embryonic development and impaired neovascularization after infarction, due to defects in endothelial cell proliferation, migration and angiogenesis.

In the context of atherosclerosis, AB are released into the circulation, promote the mobilization and incorporation of Sca-1⁺ progenitor cells during plaque formation and limit atheroprogression in a CXCR4-dependent process. Further, AB carrying miR-126 or local transfer of miR-126 reduce macrophage and apoptotic cell content but increase Cxcl12 expression and SMC content, overall resulting in smaller lesions with a less inflammatory phenotype. Notably, AB carrying miR-126 repress arterial Rgs16 expression, whereas AB deficient in miR-126 fail to reduce Rgs16 protein and to increase CXCL12 expression, establishing the mechanistic relevance of miR-126-mediated RGS16 repression *in vivo.*

## Claims

1. An isolated nucleic acid comprising a miR-126 nucleic acid sequence for use in postnatal tissue repair or for use in the prophylaxis of tissue damage.

2. The Isolated nucleic acid according to claim 1 for use in the prophylaxis or treatment of atherosclerosis.

3. The isolated nucleic acid according to claim 1 or 2, for the prophylaxis or treatment of tissue damage resulting in or due to coronary heart disease, like myocardial infarction, stroke, peripheral artery disease, aneurysm formation, or a disease related to endothelial and vascular dysfunction, in particular, pulmonary hypertension, transplant arteriopathy or various forms of vasculitis.

4. An Isolated nucleic acid comprising a miR-126 nucleic acid sequence for the recruitment of progenitor cells, in particular of Sca-1-positive progenitor cells, in particular CD31-positive progenitor cells of endothelial lineage or related to proangiogenic early-outgrowth cells.

5. The isolated nucleic acid according to any one of the preceding claims wherein said nucleic acid is miR-126 according to Seq. 1 or Seq. 2.

6. The isolated nucleic acid according to any one of the preceding claims in the form of apoptotic bodies.

7. Isolated or synthetic apoptotic bodies containing nucleic acid molecules according to any one of claims 1 to 5.

8. The isolated nucleic acid according to any one of claims 1 to 5 present in form of lipid membrane vessels, like liposomes, or polybutylcyanoacrylat (PBCA)-stabilized microbubbles.

9. The nucleic acids according to any one of claims 1 to 5 **characterized in that** the nucleic acid is for intravenous//intraarterial administration, local drug-eluting preparations including stents, and for administration per inhalation, or topical/subcutaneous administration.

10. The isolated nucleic acid according to any one of claims 1 to 5 for the prophylaxis and/or treatment of postnatal tissue damage wherein the subject to be treated is a mammal, preferably a human.

11. The nucleic acid molecules according to any one of claims 1 to 5 having furthermore comprising a vehicle for promoting site specific delivery, in particular a cholesterol-tag, or other lipid-tags or biofunctionalized (e.g. antibody—tagged) artificial particles like polybutylcyanoacrylat (PBCA)-stabilized microbubbles containing miR-126 for high-frequency ultrasound-mediated imaging and delivery.

12. Pharmaceutical composition containing a nucleic acid according to any one of claims 1 to 11.

13. The use of an isolated nucleic acid comprising a miR-126 nucleic acid sequence for recruitment of progenitor cells to a tissue.

14. The use of endothelial cell derived apoptotic bodies for use in postnatal tissue repair, in particular, for the treatment of atherosclerosis.


SEQUENCE LISTING

<110>  RWTH Aachen

<120>  Micro-RNA and tissue repair

<130>  4589-002

<160>  55

<170>  PatentIn version 3.3

<210>  1
<211>  22
<212>  DNA
<213>  human

<400>  1
ucguaccgug aguaauaaug cg                                                22

<210>  2
<211>  21
<212>  DNA
<213>  human

<400>  2
ucguaccgug aguaauaaug c                                                 21

<210>  3
<211>  17
<212>  DNA
<213>  artificial

<220>
<223>  consensus sequence

<400>  3
yayyrukasu wwurrur                                                      17

<210>  4
<211>  85
<212>  DNA
<213>  human

<400>  4
cgcuggcgac gggacauuau uacuuuuggu acgcgcugug acacuucaaa cucguaccgu      60

gaguaauaau gcgccgucca cggca                                             85

<210>  5
<211>  21
<212>  DNA
<213>  human

<400>  5
cauuauuacu uuugguacgc g                                                 21

<210>  6
<211>  21
<212>  DNA
<213>  bos taurus

<400>  6

cguaccguga guaauaaugc g                                    21

<210>  7
<211>  21
<212>  DNA
<213>  danio rerio

<400>  7
ucguaccgug aguaauaaug c                                    21

<210>  8
<211>  21
<212>  DNA
<213>  danio rerio

<400>  8
cauuauuacu uuugguacgc g                                    21

<210>  9
<211>  21
<212>  DNA
<213>  Fugu rubripes

<400>  9
ucguaccgug aguaauaaug c                                    21

<210>  10
<211>  21
<212>  DNA
<213>  Mus musculus

<400>  10
cauuauuacu uuugguacgc g                                    21

<210>  11
<211>  21
<212>  DNA
<213>  mus musculus

<400>  11
ucguaccgug aguaauaaug c                                    21

<210>  12
<211>  23
<212>  DNA
<213>  gallus gallus

<400>  12
ucguaccgug aguaauaaug cgc                                  23

<210>  13
<211>  21
<212>  DNA
<213>  gallus gallus

<400>  13
cauuauuacu uuugguacgc g                                    21

<210>  14
<211>  21

<212>  DNA
<213>  rattus norvegicus

<400>  14
ucguaccgug aguaauaaug c                                                21


<210>  15
<211>  21
<212>  DNA
<213>  rattus norvegicus

<400>  15
   cauuauuacu uuugguacgc g                                                 21


<210>  16
<211>  21
<212>  DNA
<213>  Macaca mulatta

<400>  16
ucguaccgug aguaauaaug c                                                21


<210>  17
<211>  21
<212>  DNA
<213>  Xenopus tropicalis

<400>  17
ucguaccgug aguaauaaug c                                                21


<210>  18
<211>  21
<212>  DNA
<213>  Xenopus tropicalis

<400>  18
   cauuauuacu uuugguacgc g                                                 21


<210>  19
<211>  73
<212>  DNA
<213>  bos taurus

<400>  19
ugacgggaca uuauuacuuu ugguacgcgc ugugacacuu caaacucgua ccgugaguaa      60

uaaugcgcug uca                                                         73


<210>  20
<211>  101
<212>  DNA
<213>  danio rerio

<400>  20
gagccauuuu aacugcuuca caguccauua uuacuuuugg uacgcgcuag gccagacuca      60

aacucguacc gugaguaaua augcacugug gcaguggguu u                         101


<210>  21
<211>  63
<212>  DNA

<213>  Fugu rubripes

<400>  21
cggcccauua uuacuuuugg uacgcgcuau gccacucuca acucguaccg ugaguaauaa      60

ugc                                                                   63


<210>  22
<211>  84
<212>  DNA
<213>  gallus gallus

<400>  22
gcuggugacg gcccauuauu acuuuuggua cgcgcuguga cacuucaaac ucguaccgug      60

aguaauaaug cgcugugguc agca                                            84


<210>  23
<211>  73
<212>  DNA
<213>  mus musculus

<400>  23
ugacagcaca uuauuacuuu ugguacgcgc ugugacacuu caaacucgua ccgugaguaa      60

uaaugcgcgg uca                                                        73


<210>  24
<211>  73
<212>  DNA
<213>  rattus norvegicus

<400>  24
ugacagcaca uuauuacuuu ugguacgcgc ugugacacuu caaacucgua ccgugaguaa      60

uaaugcgugg uca                                                        73


<210>  25
<211>  63
<212>  DNA
<213>  Macaca mulatta

<400>  25
cggcccauua uuacuuuugg uacgcgcuau gccacucuca acucguaccg ugaguaauaa      60

ugc                                                                   63


<210>  26
<211>  69
<212>  DNA
<213>  Xenopus tropicalis

<400>  26
ggcugugcau uauuacuuuu gguacgcgcu gugucacauc aaacucguac cgugaguaau      60

aaugcgcag                                                             69


<210>  27
<211>  16
<212>  PRT
<213>  artificial

<220>
<223>  penantrin

<400>  27

Arg Gln Ile Lys Ile Trp Phe Gln Asn Arg Arg Met Lys Trp Lys Lys
1               5                   10                  15


<210>  28
<211>  14
<212>  PRT
<213>  artificial

<220>
<223>  TAT fragment

<400>  28

Gly Arg Lys Lys Arg Arg Gln Arg Arg Arg Pro Pro Gln Cys
1               5                   10


<210>  29
<211>  27
<212>  PRT
<213>  artificial

<220>
<223>  signal sequence based peptide

<400>  29

Gly Ala Leu Phe Leu Gly Trp Leu Gly Ala Ala Gly Ser Thr Met Gly
1               5                   10                  15


Ala Trp Ser Gln Pro Lys Lys Lys Arg Lys Val
            20                  25


<210>  30
<211>  18
<212>  PRT
<213>  artificial

<220>
<223>  PVEC

<400>  30

Leu Leu Ile Ile Leu Arg Arg Arg Ile Arg Lys Gln Ala His Ala His
1               5                   10                  15


Ser Lys



<210>  31
<211>  26
<212>  PRT
<213>  artificial

<220>
<223>  transportan

<400>  31

Gly Trp Thr Leu Asn Ser Ala Gly Tyr Leu Leu Lys Thr Asn Leu Lys
1               5                   10                  15

Ala Leu Ala Ala Leu Ala Lys Lys Ile Leu
            20                  25

<210>  32
<211>  18
<212>  PRT
<213>  artificial

<220>
<223>  amphiphilic model peptide

<400>  32

Lys Leu Ala Leu Lys Leu Ala Leu Lys Ala Leu Lys Ala Ala Leu Lys
1               5                   10                  15

Leu Ala

<210>  33
<211>  9
<212>  PRT
<213>  artificial

<220>
<223>  Arg9

<400>  33

Arg Arg Arg Arg Arg Arg Arg Arg Arg
1               5

<210>  34
<211>  31
<212>  PRT
<213>  artificial

<220>
<223>  Cecropin P1

<400>  34

Ser Trp Leu Ser Lys Thr Ala Lys Lys Leu Glu Asn Ser Ala Lys Lys
1               5                   10                  15

Arg Ile Ser Glu Gly Ile Ala Ile Ala Ile Gln Gly Gly Pro Arg
            20                  25                  30

<210>  35
<211>  36
<212>  PRT
<213>  artificial

<220>
<223>  b-defensin

<400>  35

Asp His Tyr Asn Cys Val Ser Ser Gly Gly Gln Cys Leu Tyr Ser Ala
1               5                   10                  15

Cys Pro Ile Phe Thr Lys Ile Gln Gly Thr Cys Tyr Arg Gly Lys Ala
            20                  25                  30

Lys Cys Cys Lys
        35

<210>  36
<211>  30
<212>  PRT
<213>  artificial

<220>
<223>  a-defensin

<400>  36

Ala Cys Tyr Cys Arg Ile Pro Ala Cys Ile Ala Gly Glu Arg Arg Tyr
1               5                   10                  15

Gly Thr Cys Ile Tyr Gln Gly Arg Leu Trp Ala Phe Cys Cys
            20                  25                  30

<210>  37
<211>  11
<212>  PRT
<213>  artificial

<220>
<223>  Bactenicin

<400>  37

Arg Lys Cys Arg Ile Trp Ile Arg Val Cys Arg
1               5                   10

<210>  38
<211>  42
<212>  PRT
<213>  artificial

<220>
<223>  PR-39

<400>  38

Arg Arg Arg Pro Arg Pro Pro Tyr Leu Pro Arg Pro Arg Pro Pro Pro
1               5                   10                  15

Phe Phe Pro Pro Arg Leu Pro Pro Arg Ile Pro Pro Gly Phe Pro Pro
            20                  25                  30

Arg Phe Pro Pro Arg Phe Pro Gly Lys Arg
        35                  40

<210>  39

<211>  13
<212>  PRT
<213>  artificial

<220>
<223>  Indolicidin

<400>  39

Ile Leu Pro Trp Lys Trp Pro Trp Trp Pro Trp Arg Arg
1               5                   10


<210>  40
<211>  16
<212>  PRT
<213>  artificial

<220>
<223>  peptide

<400>  40

Ala Ala Val Ala Leu Leu Pro Ala Val Leu Leu Ala Leu Leu Ala Pro
1               5                   10                  15


<210>  41
<211>  11
<212>  PRT
<213>  artificial

<220>
<223>  peptide

<400>  41

Ala Ala Leu Leu Pro Val Leu Leu Ala Ala Pro
1               5                   10


<210>  42
<211>  13
<212>  PRT
<213>  artificial

<220>
<223>  peptide TAT

<400>  42

Gly Arg Lys Lys Arg Arg Gln Arg Arg Arg Pro Pro Gln
1               5                   10


<210>  43
<211>  16
<212>  PRT
<213>  artificial

<220>
<223>  peptide antennapedia

<400>  43

Arg Gln Ile Lys Ile Trp Phe Gln Asn Arg Arg Met Lys Trp Lys Lys
1               5                   10                  15

<210> 44
<211> 21
<212> DNA
<213> artificial

<220>
<223> primer aldolase

<400> 44
agctgtctga catcgctcac g 21

<210> 45
<211> 22
<212> DNA
<213> artificial

<220>
<223> primer aldolase

<400> 45
cacatactgg cagcgcttca ag 22

<210> 46
<211> 21
<212> DNA
<213> artificial

<220>
<223> primer cxcl12

<400> 46
cgccactgcc ttcacctcct c 21

<210> 47
<211> 26
<212> DNA
<213> artificial

<220>
<223> cxcl12 primer

<400> 47
ggcatacata ggcttcagag gcaatc 26

<210> 48
<211> 20
<212> DNA
<213> artificial

<220>
<223> primer RGS16

<400> 48
agggcacacc agatctttga 20

<210> 49
<211> 20
<212> DNA
<213> artificial

<220>
<223> primer RGS16

<400> 49
gtctgcaggt tcatcctcgt 20

<210> 50
<211> 22
<212> DNA
<213> artificial

<220>
<223> primer SPRED

<400> 50
tttaactaaa tgtaaggtac ga 22

<210> 51
<211> 23
<212> DNA
<213> artificial

<220>
<223> primer VCAM-1

<400> 51
tgtatagtac tggcatggta cgg 23

<210> 52
<211> 22
<212> DNA
<213> artificial

<220>
<223> primer RSG16

<400> 52
gccagtgttt tttgtggtat ga 22

<210> 53
<211> 21
<212> DNA
<213> artificial

<220>
<223> RSG16 primer mutated

<400> 53
gcgtgataat gtgaccatgc t 21

<210> 54
<211> 23
<212> DNA
<213> artificial

<220>
<223> primer cxcl12

<400> 54
tgcatttata gcatacggta tga 23

<210> 55
<211> 22
<212> DNA

<213>  artificial

<220>
<223>  primer cxcl12

<400>  55
gcgttaataa ggattgccat tt                                                22